# EUROPEAN PATENT APPLICATION

(11) **EP 4 621 395 A1**
(43) Date of publication of application: **24.09.2025**
(21) Application number: 25163388.9
(22) Date of filing: 13.03.2025
(51) Int. Cl.: G01N 23/04, G01N 23/083

(54) **X-RAY INSPECTION DEVICE**

(30) Priority: 18.03.2024 JP 2024042541
(71) Applicant: Ishida Co., Ltd., Kyoto-shi Kyoto 606-8392 (JP)
(72) Inventor: OSE, Akane, Ritto-shi, Shiga, 520-3026 (JP); SUGIMOTO, Kazuyuki, Ritto-shi, Shiga, 520-3026 (JP)
(74) Representative: Global IP Europe Patentanwaltskanzlei

(57) **Abstract**

An X-ray inspection device includes an X-ray irradiation unit configured to irradiate an article with X-rays, an X-ray detection unit configured to detect the X-rays, and an inspection unit configured to inspect the article based on an image created from a detection result of the X-rays detected by the X-ray detection unit. The inspection unit is further configured to determine that a quality of the article is low in a case where a quality index value is greater than a threshold value, the quality index value being calculated based on an area size of a region, in which the article is present, in the image and based on shading in the image or a weight of the article.

## Description

### TECHNICAL FIELD

The present disclosure relates to an X-ray inspection device.

### BACKGROUND

An X-ray inspection device is known, which includes an X-ray irradiation unit that irradiates an article with X-rays, an X-ray detection unit that detects the X-rays, and an inspection unit that inspects the article based on an X-ray transmission image created from a detection result of the X-rays detected by the X-ray detection unit. As such an X-ray inspection device, for example, Japanese Patent No. 7393793 discloses a device that determines that an article is a defective article in a case where a range of pixels in which brightness of each pixel of an X-ray transmission image is less than a first threshold value and equal to or greater than a second threshold value is equal to or larger than a predetermined range.

### SUMMARY

For example, in a case of an article such as meat, at least a portion of the article is harder than a good article, the article may be handled as an article having a low quality (hereinafter, also referred to as a "low-quality article"). From this, in the X-ray inspection device as described above, there is a demand for accurate detection of the low-quality article in addition to detection of a foreign matter mixed in the article. In particular, in recent years, there has been a problem with "woody breast", which is chicken breast that has been unnaturally hardened, and thus this is particularly pronounced.

Therefore, an object of the present disclosure is to provide an X-ray inspection device capable of accurately detecting an article of which a quality has deteriorated.

As a result of intensive studies, the present disclosers have obtained a finding that the following difference may occur between the good article and the low-quality article of which at least a portion is harder than the good article in the X-ray transmission image created from the detection result of the X-rays transmitted through the article. That is, the good article is conveyed in a state of sagging and spreading by a dead weight, and is likely to appear light and large in the X-ray transmission image. On the other hand, the present disclosers have obtained a finding that the low-quality article is conveyed in a state of a lump that appears to be contracted due to the hardness thereof, and is likely to appear dark and small in the X-ray transmission image. Therefore, the present disclosers have found that the low-quality article can be accurately detected by the X-ray inspection device based on such findings, and have reached the present disclosure.
(1) An aspect of the present disclosure relates to an X-ray inspection device including: an X-ray irradiation unit configured to irradiate an article with X-rays; an X-ray detection unit configured to detect the X-rays; and an inspection unit configured to inspect the article based on an image created from a detection result of the X-rays detected by the X-ray detection unit, in which the inspection unit is further configured to determine that a quality of the article is low in a case where a quality index value is greater than a threshold value, the quality index value being calculated based on an area size of a region, in which the article is present, in the image and based on shading in the image or a weight of the article.
   In this X-ray inspection device, it is possible to accurately detect the low-quality article by using the above-described findings that the good article is likely to appear light and large in the X-ray transmission image, and the low-quality article that is harder than the good article is likely to appear dark and small in the X-ray transmission image.
(2) In the X-ray inspection device according to (1), the inspection unit may be further configured calculate the quality index value by dividing a weight estimated based on shading in the region by the area size of the region. In this case, the above-described findings can be effectively used to detect the low-quality article with higher accuracy.
(3) In the X-ray inspection device according to (1), the inspection unit may be further configured to calculate the quality index value by dividing a weight estimated based on shading in a dark portion by an area size of the dark portion, and a density in the region is greater than a certain value in the dark portion. In this case, it is possible to accurately detect the low-quality article that partially includes a portion harder than the good article.
(4) The X-ray inspection device according to (1) to (3) may further include a detection unit configured to detect a height of the article, in which the inspection unit is further configured to calculate a volume of the article based on the height of the article detected by the detection unit and on the area size of the region, and calculate the quality index value based on the volume of the article and on shading in the image or the weight of the article. In this case, the quality index value can be calculated in consideration of the volume of the article, and the low-quality article can be detected with higher accuracy.
(5) In the X-ray inspection device according to any one of (1) to (4), the inspection unit may be further configured to determine that, in a case where the article is chicken breast, the quality of the article is low and that the article includes woody breast. In this case, it is possible to ship the chicken breast having a higher quality.

According to the present disclosure, it is possible to provide the X-ray inspection device capable of accurately detecting the article of which the quality has been deteriorated.

### BRIEF DESCRIPTION OF THE DRAWINGS

FIG. 1 is a configuration diagram illustrating an X-ray inspection device according to an embodiment.
FIG. 2 is a configuration diagram illustrating an inner portion of a shield box of FIG. 1.
FIG. 3 is a block diagram illustrating a functional configuration of a controller of FIG. 1.
FIG. 4A is a diagram illustrating an example of an X-ray transmission image related to a good article. FIG. 4B is a diagram illustrating an example of an X-ray transmission image related to a defective article.

### DETAILED DESCRIPTION

Hereinafter, an embodiment will be described with reference to the drawings. In the description of the drawings, the same elements are denoted by the same reference numerals, and the redundant description will be omitted.

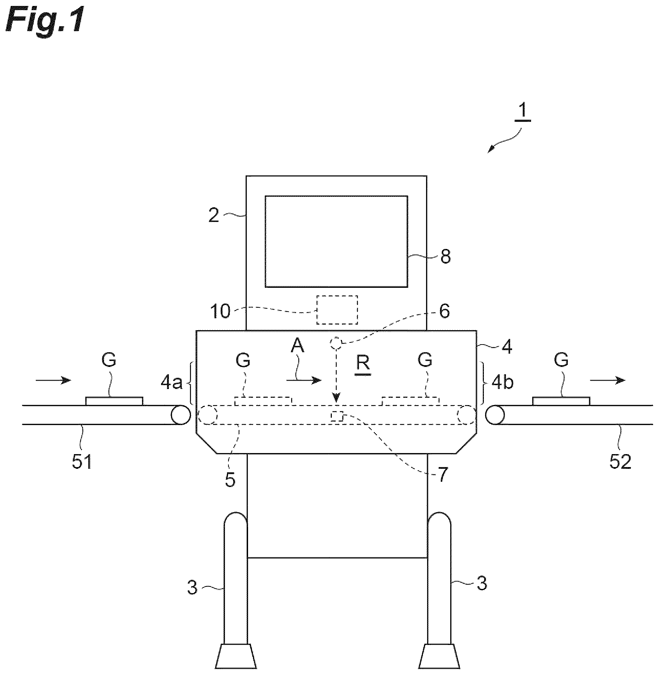

As illustrated in FIG. 1, an X-ray inspection device 1 generates an X-ray transmission image of an article G while conveying the article G, and inspects the article G based on the X-ray transmission image. The article G before the inspection is carried into the X-ray inspection device 1 by a carry-in conveyor 51. The article G after the inspection is carried out by a carry-out conveyor 52 from the X-ray inspection device 1. The article G is a product having a certain size. The article G is not particularly limited, but may include, for example, meat. In the present embodiment, the article G may include chicken breast.

The X-ray inspection device 1 includes a device body 2, a support leg 3, a shield box 4, a conveyance unit 5, an X-ray irradiation unit 6, an X-ray detection unit 7, a display operation unit 8, and a controller 10. The device body 2 houses the controller 10 and the like. The support leg 3 supports the device body 2. The shield box 4 is provided in the device body 2. The shield box 4 prevents X-rays (electromagnetic waves) from leaking to the outside. An inspection region R in which the article G is inspected by the X-rays is provided inside the shield box 4. A carry-in port 4a and a carry-out port 4b are formed in the shield box 4. The conveyance unit 5 conveys the article G. In the illustrated example, the conveyance unit 5 conveys the article G from the carry-in port 4a of the shield box 4 to the carry-out port 4b along a conveyance direction A through the inspection region R. The conveyance unit 5 is, for example, a belt conveyor.

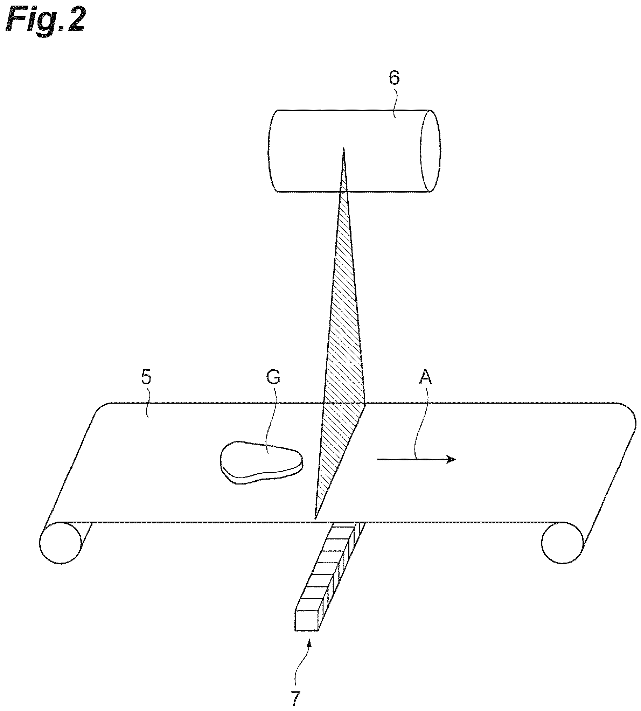

As illustrated in FIGS. 1 and 2, the X-ray irradiation unit 6 is an X-ray source that irradiates the article G conveyed by the conveyance unit 5, with the X-rays. The X-ray irradiation unit 6 is disposed in the shield box 4. The X-ray irradiation unit 6 includes, for example, an X-ray tube that emits X-rays and a stop unit that spreads the X-rays emitted from the X-ray tube in a fan shape in a plane perpendicular to the conveyance direction A. The X-rays with which the irradiation is performed from the X-ray irradiation unit 6 may include X-rays in various energy bands from low energy (long wavelength) to high energy (short wavelength).

The X-ray detection unit 7 is a sensor member that detects the X-rays transmitted through the article G irradiated with the X-rays by the X-ray irradiation unit 6. The X-ray detection unit 7 may be a line sensor or a two-dimensionally disposed sensor group. The display operation unit 8 is provided in the device body 2. The display operation unit 8 displays various types of information and receives an input operation of various conditions from the outside. The display operation unit 8 is, for example, a liquid-crystal display, and displays an operation screen as a touch panel. In this case, an operator can input various conditions via the display operation unit 8.

The controller 10 controls the operation of each unit of the X-ray inspection device 1. The controller 10 includes a processor such as a central processing unit (CPU), a memory such as a read-only memory (ROM) and a random-access memory (RAM), and a storage such as a solid-state drive (SSD). A program for controlling the X-ray inspection device 1 is recorded in the ROM. The controller 10 constitutes an inspection unit that inspects the article based on the X-ray transmission image (image) created from a detection result of the X-rays detected by the X-ray detection unit 7.

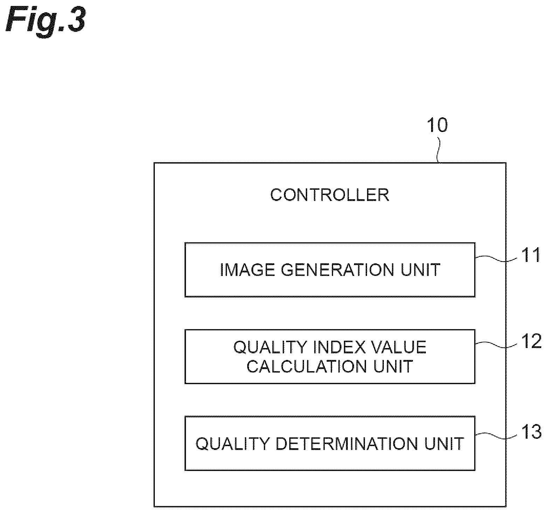

As illustrated in FIG. 3, the controller 10 includes an image generation unit 11, a quality index value calculation unit 12, and a quality determination unit 13. In the controller 10, the image generation unit 11, the quality index value calculation unit 12, and the quality determination unit 13 are configured as software. However, each of these units may be configured as hardware. The image generation unit 11 generates the X-ray transmission image of the article G based on the detection result obtained by the X-ray detection unit 7. The quality index value calculation unit 12 calculates a quality index value that is a value serving as an index of a quality of the article G, based on an area size of a region, in which the article G is present, in the generated X-ray transmission image and based on shading in the X-ray transmission image.

As an example, the quality index value calculation unit 12 obtains the area size of the region (hereinafter, also referred to as an "article region"), in which the article G is present, in the X-ray transmission image by performing image processing (binarization with a predetermined % value, and the like) on the X-ray transmission image. In addition, the quality index value calculation unit 12 estimates a weight of each pixel based on shading in each pixel in the article region of the X-ray transmission image based on the property that a heavier substance appears darker on the X-ray transmission image, and estimates the weight of the article G by integrating the weights. Then, the quality index value calculation unit 12 calculates the quality index value by dividing the weight of the article G by the area size of the article region.

In a case where the calculated quality index value is greater than a threshold value, the quality determination unit 13 determines that the quality of the article G is low. Specifically, in a case where the calculated quality index value is greater than the threshold value, the quality determination unit 13 determines that at least a portion of the article G as a quality determination target is a low-quality article that is harder than a good article (normal article). In the present embodiment, the quality determination unit 13 determines that the quality of the article G is low and that the article G includes woody breast (that is, determines that the article G is the low-quality article including the woody breast).

The threshold value may be set in advance, and stored in the controller 10. The threshold value may be set for each type of the article G. The threshold value may be a fixed value or a variable value that can be changed by a user via the display operation unit 8 or the like. The woody breast is chicken breast that has been unnaturally hardened. The woody breast is chicken breast that is hardened like wood. The woody breast is chicken breast having a gritty texture, and has a high occurrence probability in a large chicken. The woody breast is fibrous (muscular) chicken breast. The woody breast is also referred to as wood breast.

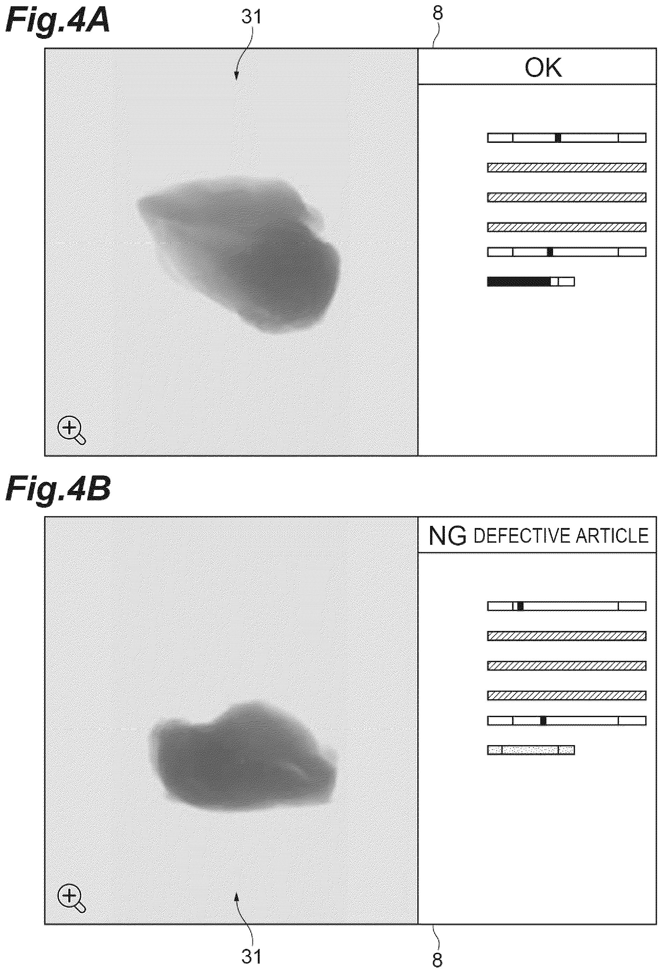

In a case where it is not determined that the quality of the article G is low as a result of the determination via the quality determination unit 13, the controller 10 causes the display operation unit 8 to display that the article G is OK (that the article G is the good article) together with the generated X-ray transmission image 31 (see FIG. 4A). In a case where it is determined that the quality of the article G is low as a result of the determination via the quality determination unit 13, the controller 10 causes the display operation unit 8 to display that the article G is NG and is a defective article (that the article G is the low-quality article) together with the generated X-ray transmission image 31 (see FIG. 4B).

Here, a finding is obtained, which indicates that the following difference may occur between the good article and the low-quality article of which at least a portion is harder than the good article in the X-ray transmission image. That is, the good article is conveyed in a state of sagging and spreading by a dead weight, and is likely to appear light and large in the X-ray transmission image. On the other hand, a finding is obtained, which indicates that the low-quality article is conveyed in a state of a lump that appears to be contracted due to the hardness thereof, and is likely to appear dark and small in the X-ray transmission image.

Therefore, in the X-ray inspection device 1, in a case where the quality index value, which is calculated based on the area size of the article region in the X-ray transmission image 31 and based on shading in the image, is greater than the threshold value, the controller 10 determines that the quality of the article G is low. As a result, it is possible to accurately detect the low-quality article by using the above-described findings that the good article is likely to appear light and large in the X-ray transmission image and that the low-quality article that is harder than the good article is likely to appear dark and small in the X-ray transmission image.

In the X-ray inspection device 1, the quality index value is calculated by dividing the weight estimated based on shading in the article region by the area size of the article region, by the quality index value calculation unit 12 of the controller 10. In this case, since the quality index value corresponds to the weight of the article G per unit area size of the article region, and the woody breast is clogged in an area smaller than the good article, the quality index value of the low-quality article including the woody breast increases. Therefore, in this case, it is possible to more accurately detect the low-quality article by effectively using the above-described findings.

In the X-ray inspection device 1, , the controller 10 determines that, in case where the article G is the chicken breast, the quality of the article G is low and that the article G includes the woody breast. In this case, it is possible to discriminate the low-quality article including the woody breast and to prevent the shipment of such a low-quality article. That is, the chicken breast having a high quality can be shipped.

Although the embodiment has been described above, the aspect of the present disclosure is not limited to the above-described embodiment.

In the above-described embodiment, the weight of the article G may be measured by a measuring device provided separately from the X-ray inspection device 1. In this case, the quality index value calculation unit 12 of the controller 10 may calculate the quality index value based on the area size of the article region in the X-ray transmission image and on the measured weight.

In the above-described embodiment, the quality index value calculation unit 12 of the controller 10 may calculate the quality index value by dividing a weight estimated based on shading in a dark portion by an area size of the dark portion, and a density in the article region of the X-ray transmission image is greater than a certain value. In this case, it is possible to accurately detect even a low-quality article that partially includes a portion (woody breast or the like) that is harder than a good article.

In the above-described embodiment, the detection unit that detects a height of the article G conveyed by the conveyance unit 5 may be further provided. In this case, the quality index value calculation unit 12 of the controller 10 may calculate a volume of the article G based on the height of the article G detected by the detection unit and on the area size of the article region, and calculate the quality index value based on the volume of the article G and based on shading in the X-ray transmission image or the weight of the article G. In this case, the quality index value corresponds to, for example, a weight per unit volume of the article G. Therefore, the quality index value can be calculated in consideration of the volume of the article G, and the low-quality article can be detected with higher accuracy.

In the above-described embodiment, in a case where the calculated quality index value is less than a lower limit threshold value (other threshold value), the quality determination unit 13 of the controller 10 may determine that the article G to be subjected to the quality determination is a low-quality article, the quality determination unit 13 of the controller 10 may determine the article G as the quality determination target is the low-quality article, such as an article including a portion that is too soft, an article having a sparse content, an article having a large amount of fatty meat, or an article having a poor balance between the lean meat and the fatty meat.

In the above-described embodiment, the controller 10 may determine whether or not the quality of the article G is low via the following separate processing. That is, the controller 10 may perform first processing of determining whether or not a foreign matter is present in the article G by using brightness of each pixel of the X-ray transmission image and a first threshold value related to a foreign matter inspection of the article G and second processing of determining whether the quality of the article G is high or low by using the brightness of each pixel of the X-ray transmission image and a second threshold value lower than the first threshold value related to the quality inspection of the article G, and determine, in the second processing, that the quality of the article G is low in a case where a range of pixels in which the brightness is less than the first threshold value and equal to or greater than the second threshold value is larger than a predetermined range. In addition, the controller 10 may perform binarization processing of the X-ray transmission image based on the brightness that is less than the first threshold value and equal to or greater than the second threshold value, acquire an area size of a region in the binarized image, and determine that the quality of the article G is low in a case where the area size of the region is equal to or greater than a predetermined area.

In this case, in a case where it is determined by the quality determination unit 13 that the quality of the article G is low and it is also determined by the above-described separate processing that the quality of the article G is low, it may be determined that the article G is the low-quality article (including the woody breast) with a very high probability. In addition, in a case where it is determined by only one of the quality determination unit 13 and the above-described separate processing that the quality of the article G is low, it may be determined that there is a high possibility that the article G is the low-quality article. In a case where it is not determined by both the quality determination unit 13 and the above-described separate processing that the quality of the article G is low, it may be determined that the article G is the good article (has a low possibility of being the low-quality article).

Each configuration in the above-described embodiment and the above-described modification examples is not limited to the above-described materials and shapes, and various materials and shapes can be applied. In addition, each configuration in the above-described embodiment and the above-described modification examples can be optionally applied to each configuration in another embodiment or modification example.

## Claims

1. An X-ray inspection device comprising:
an X-ray irradiation unit configured to irradiate an article with X-rays;
an X-ray detection unit configured to detect the X-rays; and
an inspection unit configured to inspect the article based on an image created from a detection result of the X-rays detected by the X-ray detection unit,
wherein the inspection unit is further configured to determine that a quality of the article is low in a case where a quality index value is greater than a threshold value, the quality index value being calculated based on an area size of a region, in which the article is present, in the image and based on shading in the image or a weight of the article.

2. The X-ray inspection device according to claim 1,
wherein the inspection unit is further configured to calculate the quality index value by dividing a weight estimated based on shading in the region by the area size of the region.

3. The X-ray inspection device according to claim 1, wherein
the inspection unit is further configured to calculate the quality index value by dividing a weight estimated based on shading in a dark portion by an area size of the dark portion, and
a density in the region is greater than a certain value in the dark portion.

4. The X-ray inspection device according to any one of claims 1 to 3, further comprising:
a detection unit configured to detect a height of the article,
wherein the inspection unit is further configured to:
calculate a volume of the article based on the height of the article detected by the detection unit and on the area size of the region, and
calculate the quality index value based on the volume of the article and on shading in the image or the weight of the article.

5. The X-ray inspection device according to any one of claims 1 to 4,
the inspection unit is further configured to determine that, in a case where the article is a chicken breast, the quality of the article is low and that the article includes woody breast.
